# EUROPEAN PATENT APPLICATION

(11) **EP 0 878 547 A1**
(43) Date of publication of application: **18.11.1998**
(21) Application number: 97201422.9
(22) Date of filing: 12.05.1997
(51) Int. Cl.: C12N 15/86, C12N 7/01, A61K 48/00

(54) **Methods for producing retroviruses with an altered tropism**

(71) Applicant: ACADEMISCH MEDISCH CENTRUM AMSTERDAM, 1105 AZ Amsterdam (NL)
(72) Inventor: The designation of the inventor has not yet been filed
(74) Representative: Smulders, Theodorus A.H.J., Ir.

(57) **Abstract**

The invention relates to the field of molecular biology and virology. It particularly relates to the field of genetic engineering techniques and the use of these techniques in preparing novel pharmaceuticals, in particular in the field of gene therapy and to methods for producing novel retroviral vectors, these vectors and recombinant viruses obtainable by the methods.

The invention provides a method for producing a retrovirus or a recombinant virus derived therefrom having an altered tropism, whereby the genome of the virus is modified so that at least one of its translation initiation sites is at least partially functionally inhibited.

The invention also provides a method for producing a recombinant vector encoding a recombinant retrovirus having modified tropism, comprising culturing said retrovirus in a host cell to allow for the virus to circumvent an introduced inhibition, identifying the sequence which was mutated to circumvent said inhibition and introducing the mutation in another vector based on a retrovirus or provirus of a retrovirus.

## Description

The present invention relates to the field of molecular biology and virology. It particularly relates to the field of genetic engineering techniques and the use of these techniques in preparing novel pharmaceuticals, in particular in the field of gene therapy. It also relates to methods for providing tools for making novel pharmaceuticals.

In particular the present invention relates to the field of retroviruses, more specifically immunodeficiency viruses such as HIV (human immunodeficiency virus) and SIV(simian immunodeficiency virus).

Currently an enormous research effort takes place all around the world in order to elucidate the life cycle of HIV, how it infects cells, which cell types it can infect, how it replicates, etc. The present invention was made during research directed at the elucidation of the initiation of translation of HIV mRNA.

During this research a solution was provided to a problem encountered in a somewhat different field, namely that of delivery of nucleic acid molecules to host cells, in particular in gene therapy protocols.

In trying to deliver nucleic acids to host cells, in particularly in gene therapy settings, a problem that often arises is that there is no delivery vehicle available which is capable of delivering the desired nucleic acid to the interior of the target host cell. Often laboratory techniques, such as electroporation and other transduction means are not available in these settings, so that delivery through transfection seems to be the method of choice.

However, the viruses used to transfect host cells and thus deliver the nucleic acid of interest to said host cell are usually either capable of transfecting any cell, or are only capable of infecting a very limited subset of cells in a population. Therefore the choice is either to infect very many non-target cells with the gene delivery vehicle (which herein is used as a term for a recombinant virus particle or the nucleic acid within such a particle, or the vector itself, which vector comprises the nucleic acid to be delivered to the target cell and which vector further is provided with a means to enter said cell, which means may be e.g. a viral capsid), or not being able to deliver the nucleic acid of interest to the desired target cells.

To overcome this problem it has been suggested to provide gene delivery vehicles with molecules on their surface, which recognize molecules on the surface of target cells and hoping that the vehicle will be internalized. However, it is known from the field of cancer imaging and tumor therapy that molecules which recognize targets associated with cells are hardly ever specific for the target cell. Therefore this approach seems rather limited in its applicability.

During our research into the translation initiation mechanism of certain retroviruses, in particular SIV and/or HIV we have found a method for producing retroviruses of which the tropism (meaning the range of target cells that can be infected by the virus) can be manipulated. Hereunder a part of the study carried out into the mechanism of translation initiation in retroviruses is given. This part is intended to illustrate which finding lies at the basis of the present invention.

The untranslated leader region of human and simian immunodeficiency virus (HIV and SIV) RNA genomes encodes multiple sequence elements that are important for virus replication (reviewed in reference 5). The approximately 300-to-400-nt-long leader contains elements involved in transcription of the provirus (trans-acting responsive TAR element), processing of the primary transcript (e.g., major splice donor site [SD]), and dimerization and packaging of the genomic RNAs. Furthermore, sequences involved in reverse transcription (e.g., primer-binding site, repeat region) are encoded by the leader RNA. Several of these replicative signals adopt a specific RNA secondary structure. For instance, TAR folds into an extended stem-loop structure, and similar hairpin motifs have been reported to mediate viral RNA packaging and dimerization.

Because the HIV-1 leader is such a complicated element, involving strong secondary structure, it is questionable whether the regular scanning mechanism is operating for translation of HIV-1 mRNAs.

Translation of eukaryotic mRNAs is currently best explained by the scanning mechanism (34). The 40S ribosomal subunit binds to the 5' cap structure of the mRNA and scans until an AUG start codon in an appropriate sequence context is encountered. A consensus sequence for efficient initiation of translation, CC(A/G)CCAUGG, has been postulated (31).

Scanning of the ribosome along the RNA can be inhibited by stable secondary structures (32,33). The HIV-1 leader RNA has two relatively stable stem-loop structures at the 5'end (the TAR and polyA hairpins), and because the two hairpins are adjacent it is possible that this structure could further stabilize by coaxial stacking of the two stems (5).

Introduction of the HIV-1 leader sequences at the 5' end of a reporter protein mRNA was found to inhibit translation in in vitro translation extracts and *Xenopus* oocytes (20,24,39,41). The TAR hairpin contains the 5'-capped nucleotide and may reduce the accessibility of the cap structure to the initiation factor eIF-4B (39). In addition, dimerization of retroviral RNA occurs in the leader region and may reduce the translational efficiency, as described previously for Rous sarcoma virus (12). These combined arguments suggest that a modified scanning mechanism, or an unrelated translation mechanism, may be operating for HIV-1.

Alternative translational strategies to bypass strong structures in the leader RNA have been described for other retro-elements. First, a process termed "ribosome shunt" has been proposed for the Cauliflower mosaic virus (23). According to this mechanism, ribosomes enter at the cap-site and begin scanning, but they are somehow transferred to a region at the 3'end of the leader. This non-linear scanning mechanism bypasses stable RNA structures in the central portion of the leader. Second, the extremely long leader of several retroviruses, including Moloney murine leukemia virus and Harvey murine sarcoma virus, was demonstrated to facilitate cap-independent ribosome binding to a motif in the 3'end of the leader, with subsequent scanning to the first AUG codon (10,11,44). This mechanism uses a socalled internal ribosome entry site (IRES) and has also been described for hepatitis C virus and several picornaviruses, including poliovirus, foot-and-mouth disease virus and hepatitis A virus (3,27).

To study the mechanism of HIV-1 mRNA translation, we introduced an optimal translation initiation site in the leader region of the viral RNA. This mutation was positioned upstream of the major splice donor site and opened a short open reading frame in both unspliced and spliced forms of viral RNA. According to the linear scanning model, the new start codon will usurp most scanning ribosomes. Upon translation of the upstream open reading frame, the ribosomes will terminate translation and dissociate from the mRNA, thus reducing the expression of all downstream viral genes. Conform the scanning mechanism, this upstream AUG mutation severely reduced the level of protein synthesis and virus replication. Upon prolonged culturing of the mutant virus, phenotypic revertants were obtained with increased replication capacity. Surprisingly, revertants obtained in three independent experiments had not inactivated the introduced start codon. Detailed analysis of one of these revertant genomes showed that two amino acid substitutions in the viral envelope (Env) protein are responsible for increased replication.

However, a further observed phenomenon in these phenotypic revertants is that the capability to infect other cells than the cell type in which the virus was cultured for a term sufficient to allow for reversion, is significantly decreased. Apparently the virus increases its capability to replicate in this host cell, but at the same time it sacrifices the capability to infect other cells. Thus it becomes possible by introducing an upstream efficient translation initiation site comprising an AUG-codon (or another mutation which cannot easily be undone) to manipulate the host range specificity of a virus by culturing it in the cell type that is desired as a target for retroviral-mediated gene delivery. Of course there must be a minimal capacity to replicate in said cell. Once a mutation circumventing the inhibition of the translation initiation site has occurred, and the mutation reponsible for phenotypic reversion has been identified, this mutation can be provided in viruses or viral vectors not having e.g. the upstream AUG mutation. Thus the tropism of these vectors can also be manipulated.

The invention provides in one embodiment a method for producing a retrovirus or a recombinant virus derived therefrom having an altered tropism, whereby the genome of the virus is modified so that at least one of its translation initiation sites is at least partially functionally inhibited.

As explained above by modifying the leader region of retroviruses, in particular SIV and/or HIV or the leader region of viral vectors based on these viruses, a mutant is made which is to a certain extent less capable of replication. Upon long term culturing in a host cell phenotypic reversion occurs, whereby the tropism of the virus or the vector is altered, in the sense that replication capability is enhanced in that particular host cell, but the capability to infect other host cells is reduced. This mechanism has proven to work in at least retroviruses in which the mechanism of translation initiation is the so-called scanning mechanism. However in other retroviruses similar phenotypic reversion may be found upon the introduction of a mutation which the virus is unable to undo and which has an effect on replication capability. Anyway the scanning mechanism probably operates in SIV and HIV as disclosed herein and these viruses or vectors based on these viruses are preferred to be used in the present invention.

A modification of the leader region which has been shown not to be undone by the virus but to be circumvented is the introduction of a translation initiation start site upstream of a regular translation initiation start site. This modification is thus preferred because it certainly leads to phenotypic revertants with an altered tropism.

Preferably the modification comprises a bit more than just a start codon AUG. It should be followed by a short open reading frame and be a very efficient translation initiation site to make sure that scanning ribosomes recognize this modified sequence as a start site. An optimal initiation site to be introduced is disclosed herein. The invention further provides a method which comprises infecting host cells with the modified virus and culturing said infected cells for a sufficient time for the modified virus to circumvent the inhibition introduced by the modification and thereby alter its tropism. Preferably the alteration of tropism is a narrowing of the host cell range accompanied by an enhancement of the infectiousness of the modified virus for a certain subset of host cells.

The invention further provides a modified retrovirus or a viral vector derived therefrom obtainable by a method according to the embodiments disclosed above. Preferably the virus is further developed to be made into a vector, comprising a nucleic acid sequence within its genome which can be used for gene therapy approaches. Preferably development is taken further to a stage where said vector is adapted to be packaged in a packaging cell line in which structural proteins of the virus are encoded by the packaging cell and no longer by the virus. This safety protocol will allow the production of replication-deficient viruses that can be used for a single-round infection (transduction) of the appropriate target cell.

Retroviral infection can occur via two mechanisms, as cell-free virus or via cell-cell contact. Both mechanisms are related in that they use the viral Env molecule, but differences are expected at the molecular level. Wherease HIV spreads in tissue culture predominantly via cell-cell transmission, a retroviral gene therapy vector will use exclusively the cell-free route. To improve HIV-based vectors, we include HIV-1 adaptation experiments to select for variants with improved 'cell-free infection capacity'.

This selection can be performed by repeated cell-free passage of the virus onto a particular target cell type.

Usually the nucleic acid sequence to be used in gene therapy will be a heterologous nucleic acid sequence of therapeutic interest.

Preferably such a sequence encodes a protein or an active nucleic acid molecule. Such proteins include, but are not limited to the wild-type form of a human protein to restore a genetic defect, a mutant, trans-dominant form of a microbial protein to treat an infectious disease, or the addition of tumor-suppressor proteins to prevent development of cancer.

Therapeutic nucleic acids include sense transcripts (decoys), antisense transcripts and ribozymes, and can be used to treat a variety of diseases, in particular infectious diseases.

Since retroviruses are RNA viruses and genetic engineering techniques are usually carried out using DNA, the invention also provides a recombinant vector for preparing a modified virus according to the invention, comprising a provirus DNA sequence having a modification resulting in an at least partial inhibition of at least one translation initiation site of the original retrovirus genome.

The preferences expressed for the vectors disclosed above apply here as well.

The modified viruses can be used in adaptation studies concerning retroviruses, in particular HIV, but it is preferred for the present invention to use the viruses or vectors based on these viruses as vehicles for the delivery of nucleic acids of interest to host cells. It is particularly preferred to use the viruses and vectors provided by the present invention in gene therapy.

As mentioned briefly hereinbefore, it is also possible to produce a modified virus using the methods disclosed above, but not the virus directly resulting from said methods (the phenotypically revertant virus), but instead to identify which mutation has occurred in the phenotypic revertant and introduce said mutation into another retrovirus, provirus or retroviral vector, so that the replication efficiency of the phenotypic revertant is provided to another virus, including the modification of the host cell range.

Thus the invention provides in a further embodiment a method for producing a recombinant vector encoding a recombinant retrovirus having modified tropism, comprising subjecting a retrovirus to a method as disclosed above, including culturing said retrovirus in a host cell to allow for the virus to circumvent the introduced inhibition, identifying the sequence which was mutated to circumvent said inhibition and introducing said mutation in another vector based on a retrovirus or provirus of a retrovirus.

It is preferred that the other retrovirus provided with the altered tropism of the phenotypic revertant is not subjected to a method for altering its tropism according to the invention, at least not before it has been provided with the mutation of the phenotypic revertant. After it has been provided with said mutation, it may be advantageous to subject the obtained virus to a second round of long term culturing in a method according to the invention.

Of course the virus or vector or provirus provided with the mutation again preferably contains a heterologous sequence, particularly one coding for a protein or an antisense nucleic acid, preferably one useful in gene therapy and preferably the virus or vector or provirus is again dependent on a packaging cell.

The invention will be explained in more detail in the following experimental part.

### EXPERIMENTAL.

### Materials and Methods

### Cells and viruses.

SupT1 T cells were grown in RPMI 1640 medium containing 10% fetal calf serum (FCS) at 37^{o}C and 5% C02 Cells were transfected with HIV-1 molecular clones by electroporation.

Briefly, 5 x 10⁶ cells were washed in RPMI with 20% FCS, mixed with 1-5 µg DNA in 0.4 cm cuvettes and electroporated at 250 Volts and 960 uF, and then resuspended in RPMI with 10% FCS. Cell were split 1 to 10 twice a week. For the selection of revertant viruses, SupT 1 cells were transfected with 5 µg DNA, and cultures were maintained up to 110 days. When HIV-induced cytopathic effects were apparent, high level virus replication was maintained by passage of the cell-free culture supernatant onto uninfected SupT1 cells. Initially, we used 50 µl to infect 10 ml SupT1 cells, but we gradually used less culture supernatant per passage (minimally 0.1 µl). Cell and supernatant samples were taken at each passage and stored at -70°C.

C33A cervix carcinoma cells (ATCC HTB31)(2) were grown as a monolayer in Dulbecco's modified Eagles medium (DMEM) supplemented with 10% FCS and MEM nonessential amino acids at 37°C and 5% CO₂. C33A cells were transfected by the calciumphosphate method. Cells were grown in 20 ml culture medium in a 75 cm²- flask to 60% confluency. 40 µg DNA in 880 µl water was mixed with 1 ml 50 mM HEPES (pH 7.1), 250 mM NaCl, 1.5 mM Na₂HPO, and 120 µl 2 M CaCl₂, incubated at room temperature for 20 min, and subsequently added to the culture medium. The culture medium was changed after 16 h.

### Mutation of the HIV-1 genome.

The full-length molecular HIV-1 clone pLAI (40) was used to produce wild-type and mutant viruses. Unless stated otherwise, nucleotide numbers refer to the position in the proviral DNA of pLAI, with position 1 being the 5' most nucleotide of the 5' long terminal repeat (LTR), and transcription starting at position 455. For comparison with the LAI genomic RNA sequence (as published in the GenBank Database, accession number K02013) one should subtract 454 from this position number. A sequence difference was found between the pLAI molecular clone that we used and the published sequence at positions 7868-7869 (GC instead of the reported sequence CG), changing amino acid 538 from Arginine (R) to Alanine (A). Both the nucleotide sequence and the amino acid sequence of pLAI at this position are now conform the consensus sequence for HIV-1 type B (38).

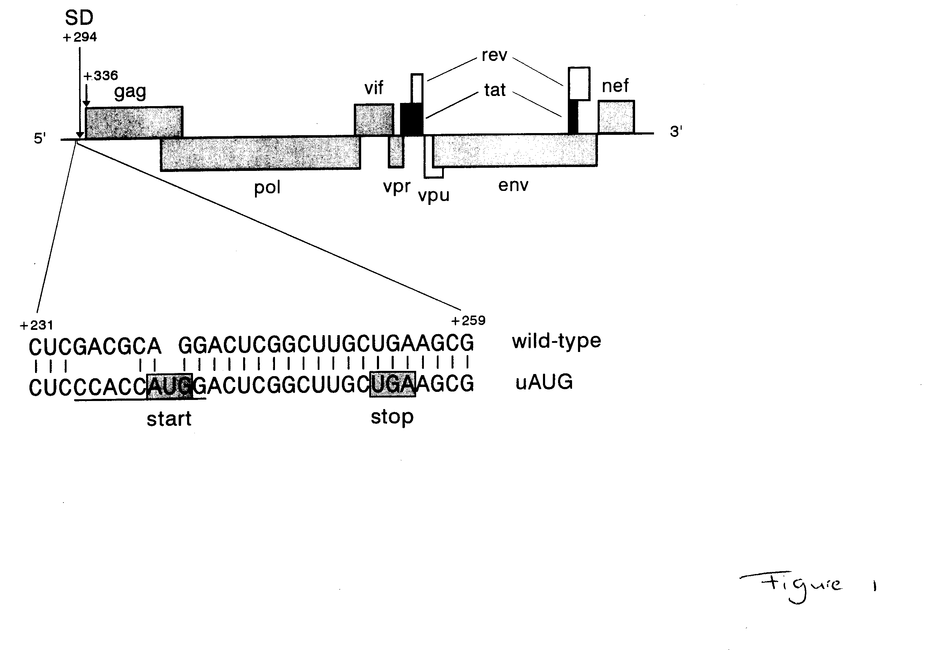

For mutation of the leader region, an *Xba*I-*Cla*I fragment encompassing the complete 5' LTR, untranslated leader and 5'-part of the *gag* gene (nucleotides 1-830) was cloned in pbluescriptII KS⁺ (Stratagene) as described previously (Blue-5'LTR)(6). The leader sequence was modified by oligonucleotide-directed in vitro mutagenesis (35) with the Muta-Gene In Vitro Mutagenesis Kit (Bio-Rad), single-stranded (minus-strand) DNA and a plus-strand mutagenic oligonucleotide: GGAGCTCTCTC**CCAC**CA**T**GGACTCGGCTT (mismatch nucleotides in bold and underlined, insertion nucleotide in bold and double underlined). This UAUG mutation was verified by sequence analysis. The mutant *Xba*I-*Cla*I fragment was introduced subsequently into the 5' LTR region of pLAI to generate the uAUG molecular clone (Fig. 1).

### Proviral DNA analysis and cloning of revertant sequences in HIV-1 proviral clones.

HIV-1 infected cells (approximately 1 x 10⁶) were pelleted by centrifugation at 4000 rpm for 4 min and washed with phosphate-buffered saline (10 mM Na-phosphate pH 7.4, 150 mM NaCl). Cells were lysed in 10 mM Tris-HCl (pH 8.0), 1 mM EDTA, 0.5 % Tween 20, and then incubated with 200 µg Proteinase K per ml at 56°C for 30 min, and heat-inactivation at 95°C for 10 min. Proviral DNA sequences were PCR-amplified from solubilized cellular DNA with P*fu* polymerase (Stratagene). Five primer sets were used to amplify different parts of the HIV-1 genome: fragment 1, 5' U3 region primer LA15'X (positions 1-20,) and 3' *gag* primer AD-GAG (896-917); fragment 11, *5' gag* primer GAGI (796-818) and 3' *pol* primer 184PMA-V (3138-3159); fragment III: 5' *pol* primer RTout5'(2413-2443) and 3' *tat/rev* primer WK533 (6048-6074); fragment IV, 5' *vpr* primer 5'env-N (5708-5736) and 3' *env/rev* primer 4TRN (8608-8628); fragment V, *5' env* primer L3 (7841-7861) and 3' U5 region primer LAI3'A (9746-9767).

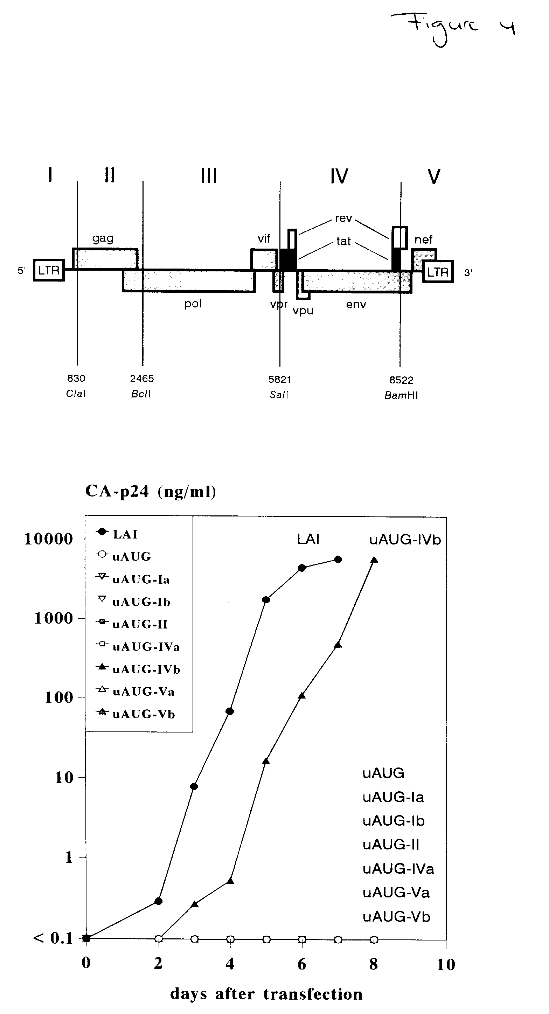

The PCR fragments were digested with restriction enzymes (fragment I, *Xba*I and *Cla*I; fragment II, *Cla*I and *Bcl*I; fragment III, *Bcl*I and *Sal*I; fragment IV, *Sal*I and *Bam*HI; fragment V, *Bam*HI and *Aat*lI; see Fig. 4 for the positions of these sites) and cloned into the uAUG proviral clone. The *Sal*I-*Bam*Hl fragment IV of revertant clone uAUG-lVb was introduced into the wild-type LAI clone (resulting in clone LAI₍ₑₙᵥ₎), the TAR-mutated Xho+10 clone (Xho+10₍ₑₙᵥ₎; 29) and the polyA hairpin mutants B and B200 (B₍ₑₙᵥ₎ and B200₍ₑₙᵥ₎; 15).

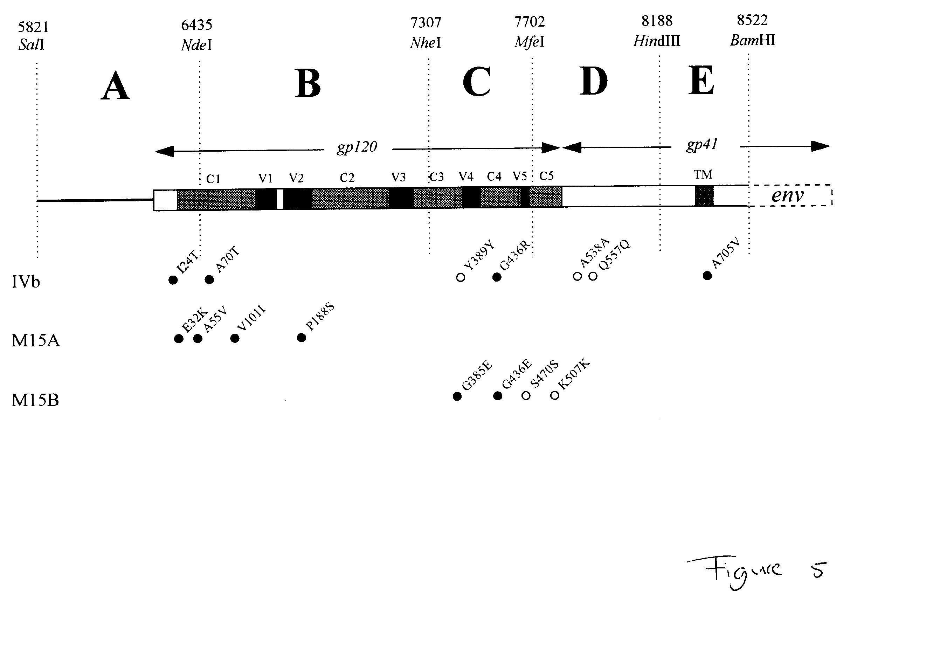

The *Sal*I-*Bam*HI fragment IV of revertant uAUG-IVb was subcloned into pBluescript and sequenced. Five subfragments were obtained (fragment A, *Sal*I and *Nde*I; B, *Nde*I and *Nhe*I; C, *Nhe*I and *Mfe*I; D, *Mfe*I and *Hind*III; E, *Hind*III and *Bam*HI; see Fig. 5 for the positions of these sites). These subfragments were used to replace the corresponding sequences in the wild-type fragment IV. The resulting *Sal*I-*Bam*HI fragments were used to replace the corresponding sequences in the uAUG proviral clone.

Two additional cultures of the uAUG mutant were maintained long-term to select for revertant viruses (M15A and M15B).

For sequencing of the *env* gene of these revertants, and for sequencing the 5'LTR-leader region of all revertants, proviral DNA sequences were PCR-amplified from total cellular DNA with AmpliTaq (Perkin Elmer). PCR fragments were cloned directly into the TA cloning vector pCRII (Invitrogen). All sequence analyses were performed with the Taq DyeDeoxy Terminator cycle sequencing protocol (Applied Biosystems) on an Applied Biosystems 373 DNA sequencer.

**Western blot analysis.** C33A cells were transfected with the proviral clones. At three days posttransfection, cells were trypsinized and collected by low speed centrifugation (1500 rpm, 10 min). Pelleted cells were washed with phosphate-buffered saline and resuspended in reducing SDS sample buffer (50 mM Tris-HCI pH 7.0, 2% SDS, 10% β-mercaptoethanol, 5% glycerol). Proteins were resolved in a 10% SDS-polyacrylamide gel, transferred to Immobilon-P (16 h, 60 V), and subsequently blocked with phosphate-buffered saline containing 5% non-fat dry milk, 3% BSA and 0.05% Tween 20. Filters were incubated with serum of an HIV-1 infected individual (patient H) for 1 h at room temperature, washed, incubated with goat anti-human IgG-alkaline phosphatase conjugate (Bio-Rad) and developed with the 5-bromo-4-chloro-3indolylphosphate/nitroblue tetrazolium protocol (Sigma).

**CA-p24 ELISA and reverse transcriptase (RT), activity** assay. CA-p24 levels were determined by ELISA (Abbott).

RT assays were performed as previously described (45). Each reaction contained 10 µl of virus-sample and 50 µl RT buffer (60 mM Tris-HCl pH8.0, 75 mM KCl. 5 mM MgCl₂, 0.1% Nonidet P-40, 1mM EDTA, 4mM DTT) supplemented with 0.25 µg polyA and 8 ng oligo(dT)18 primer and 0.1 µl [α-³²P]dTTP (3000 Ci/mmol, lOmCi/ml). After 2 h at 37°C, 10 µl was spotted onto DE-81 paper (Whatman), which was washed three times in 5% Na₂HPO₄ twice in ethanol and air-dried. RT activity was measured in the linear range of the assay and radioactive spots were quantitated on a Molecular Dynamics PhosphorImager.

### Results

### Reduced gene expression and replication of HIV-1 viruses with an upstream translation start site.

Translation of mRNAs- by ribosomal scanning is sensitive to the introduction of an open reading frame upstream of the normal translation start site. To study the translational mechanism of HIV-1 mRNAs, we introduced an additional start codon in the leader region of the HIV-1 LAI genome (Fig. 1).

This mutant, termed uAUG (upstream AUG), was designed with a minimum of nucleotide changes to create a start codon with optimal sequence context (CCACCAUGG, 31) at position +239 of the HIV-1 leader. The reading frame opened by this upstream AUG is terminated at the UGA stop codon located 12 nucleotideg downstream. Because the uAUG mutation is positioned upstream of the major splice donor site (SD at position +294; Fig. 1), each HIV-1 subgenomic mRNA species will contain this additional open reading frame. Thus, translation of all HIV-1 proteins is expected to be affected if ribosomes use the scanning mechanism.

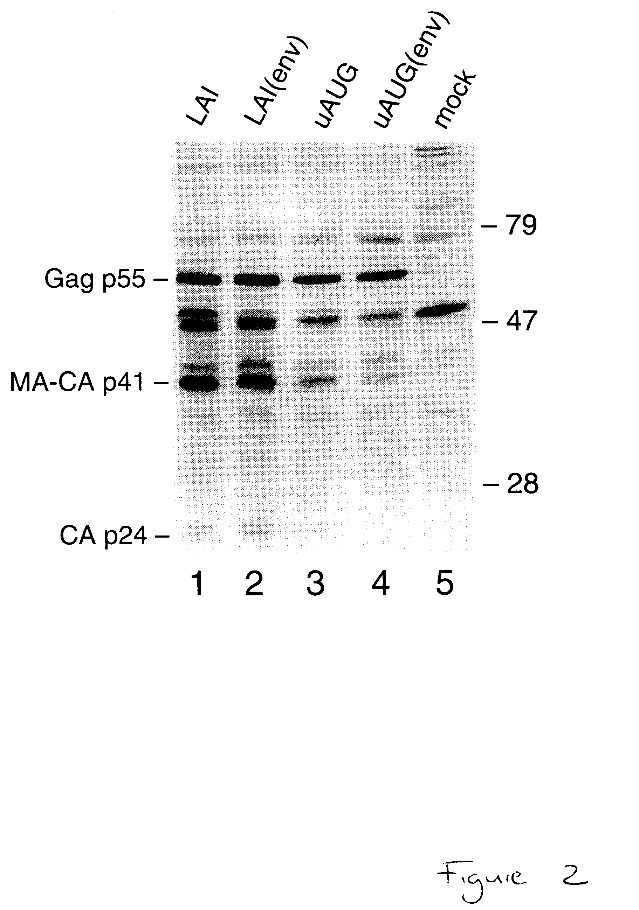

To study the effect of the leader mutation on HIV-1 protein expression, we transiently transfected the human cervix carcinoma cell line C33A with the wild-type (pLAI) and mutant (uAUG) proviral constructs. The intracellular protein levels were analyzed by Western-blotting at three days post-transfection. The production of viral proteins is severely reduced for the uAUG mutant (Fig. 2; compare lanes 1 and 3). To assay virus production, we measured CA-p24 protein and RT enzyme activity in the culture supernatant (Table 1). These levels were reduced to 11% and 18% of the wild-type control. Thus, the introduction of an upstream open reading frame strongly inhibits HIV-1 gene expression, consistent with the scanning mechanism of mRNA translation.

The replication potential of wild-type (pLAI) and mutant (uAUG) virus was measured upon transfection of the molecular clones into SupT1 cells. These T-cells express the CD4 receptor and are fully susceptible for virus replication.

Virus production was monitored by measuring CA-p24 levels in the culture medium at several times after transfection.

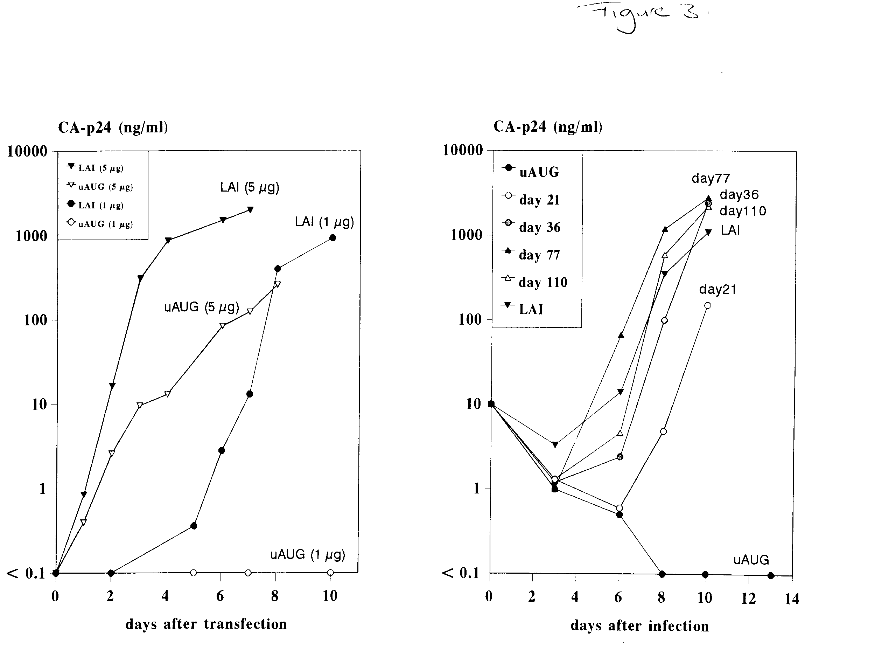

Upon transfection of 1 µg of the proviral DNAS, replication of the wild-type virus was observed within one week (Fig. 3, left panel). In contrast, no detectable level of virus production was observed for the uAUG mutant. Upon transfection of 5 µg of proviral DNA, virus production observed at day 1 and 2 results predominantly from transcription and translation of the transfected proviral DNA, whereas the further increase in virus concentration at subsequent days results from virus replication. Both transient virus production and virus replication were found to be reduced for the uAUG mutant compared with the wild-type virus. Thus, both HIV-1 gene expression and virus replication are significantly reduced by the uAUG mutation.

### Increased replication of the UAUG mutant by acquisition of second-site mutations within the env gene.

The poorly replicating uAUG mutant was cultured for up to 110 days and virus and cell samples were taken at several times. The replication capacity of these virus samples was assayed by infection of fresh SupT1 cells (Fig. 3, right panel). Viruses present at day 21 posttransfection did show improved replication kinetics compared with the replication-incompetent uAUG mutant. Upon prolonged culturing, the replication capacity of these mutants increased further to approximately wild-type levels.

It was anticipated that the phenotypic reversion was caused by inactivation of the start codon that we had introduced in the leader region. To determine the sequence of the leader region of these phenotypic revertants, total cellular DNA was isolated from the infected cells that were sampled at different times of the prolonged culture. The complete 5' LTR-leader region of proviral DNA was PCR-amplified and cloned, followed by sequencing of multiple clones of each sample. Surprisingly, we did not observe nucleotide changes in or near the short open reading frame in the leader region. We therefore performed two additional reversion experiments in which the uAUG mutant was cultured for up to 190 and 151 days (cultures M15A and M15B, respectively).

During this period the amount of virus necessary to infect fresh SupT1 cells gradually decreased, indicating an improvement of the viral replication capacity. Consistent with the results obtained in the initial experiment, we found no sequence changes in or near the upstream open reading frame of these revertant viruses.

These results indicate that sequence changes elsewhere in the HIV-1 genome are responsible for the increased replication capacity of the uAUG revertants. To identify these second-site mutations, we PCR-amplified five proviral DNA fragments (Fig. 4, fragments I-V) of the day 110 sample of the initial reversion experiment (Fig. 3). These PCR fragments were used to replace the corresponding sequences of the original uAUG clone. The replication potential of the uAUG-revertant clones was then assayed by transfection of SupT1 cells. Upon transfection of 1 µg of DNA, only revertant clone uAUG-lVb demonstrated efficient replication, whereas the uAUG mutant and all other uAUG-revertant clones did not produce detectable amounts of virus (Fig. 4, bottom panel). In clone uAUG-IVb the nucleotides from position 5821 to 8522 had been replaced by PCR-amplified proviral sequences. Although the same *Sal*I-*Bam*HI fragment was replaced in revertant clone uAUG-IVa, this recombinant virus did not show improved replication. This may be caused by the introduction of deleterious mutations during PCR amplification of the 2.7 kb revertant sequences. We were unable to clone and test fragment III, which encodes most of the *pol* gene, and decided to focus on fragment IVb that improved the replication capacity of the uAUG virus.

The fragment exchanged in revertant uAUG-IVb contains part of the *env*, *vpr* and *rev* open reading frames and the complete *tat* and *vpu* genes. Sequencing of this fragment revealed three silent and four non-silent nucleotide changes in the *env* region (Fig. 5). The 5' most mutation, which generates the 124T substitution in Env, encodes a silent mutation in the overlapping *vpu* open reading frame. Sequencing of the *env* regions of revertant viruses in the cultures M15A and M15B also revealed several sequence changes (Fig. 5). In the M15A virus four non-silent mutations were located in the 5' *env* region, whereas in the M15B virus two nonsilent and two silent mutations were found in the middle part of the *env* gene. No identical amino acid substitutions were observed among the three independent reversion experiments, except that Glycine 436 in the Env C4 domain was replaced in two cultures (G436R in IVb and G436E in M15B).

### Envelope mutations do not specifically overcome the gene expression defect of the uAUG mutant, but improve virus replication.

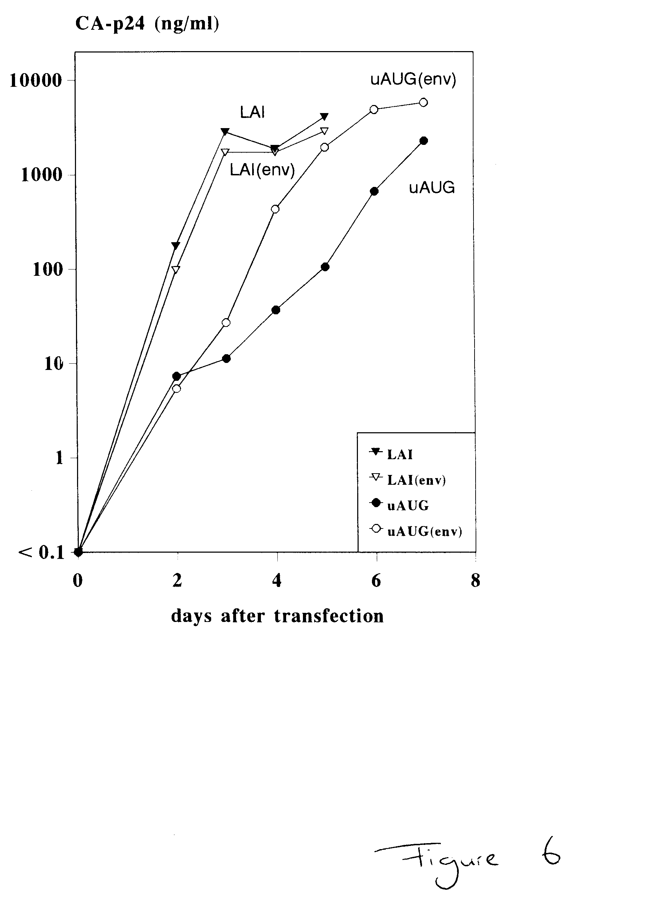

As mentioned above, the uAUG mutation reduced the production of viral proteins in transfected cells. We therefore tested whether the *env* sequence of revertant IVb could restore protein expression of the uAUG mutant. We will refer to this uAUGIVb construct as uAUG₍ₑₙᵥ). Upon transient transfection C33A cells, a similar production of viral proteins (Fig. 2) and viruses (Table 1) was observed for the the uAUG and uAUG₍ₑₙᵥ₎ clones. Thus, introduction of the env mutations did not repair the protein expression defect of the uAUG mutant. Similar results were obtained by transfection of 5 µg of the proviral clones into SupT1 cells. The virus concentration measured at 2 days posttransfection, which results predominantly from transcription and translation of the transfected DNA, was not increased by the *env* mutations (compare uAUG and uAUG₍ₑₙᵥ₎ in Fig. 6). Upon prolonged culturing, more efficient replication of the uAUG₍ₑₙᵥ₎ virus was observed.

Thus, the *env* mutations do not directly overcome the gene expression defect of the uAUG mutant, but improve some other step in the replication cycle of the virus.

The revertant *env* sequences were also introduced into the wild-type HIV-1 molecular clone LAI, producing LAI₍ₑₙᵥ₎.

The revertant *env* sequences did not affect protein and virus production of the wild-type virus in C33A cells (Fig. 2 and Table 1; compare LAI and LAI₍ₑₙᵥ₎). Furthermore, the *env* mutations did not improve virus production and replication of the wild-type virus (LAI) in SupT1 cells (Fig. 6).

Apparently, replication of the wild-type LAI virus is optimal in these cells and cannot be improved by introduction of the modified Env protein.

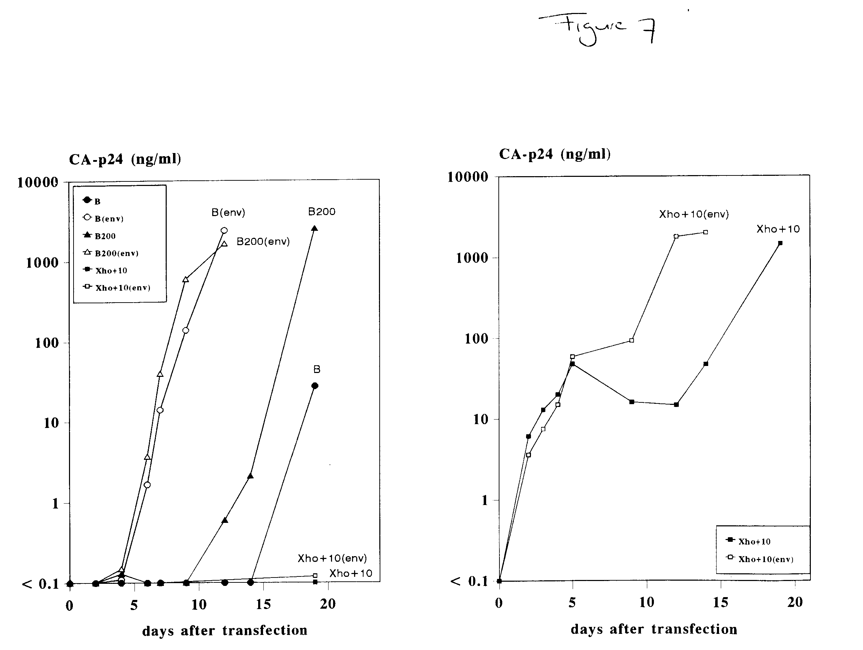

Because the *env* adaptations did not overcome the specific defect of the UAUG mutant, the modified Env protein can also improve replication of other replication impaired HIV-1 mutants. For this reason, the *SaI*l-*Bam*HI fragment of uAUG₍ₑₙᵥ₎ was introduced into mutant HIV-1 clones altered in either the TAR hairpin (mutant Xho+10; 29) or the polyA hairpin motif (mutants B and B200; (15). These mutants are defective in transcription and RNA packaging, respectively (15,29). As shown in Fig. 7, replication of all mutants was significantly increased by the revertant *env* sequences (compare B with B₍ₑₙᵥ₎, B200 with B200₍ₑₙᵥ₎ and Xho+10 with Xho+10₍ₑₙᵥ₎). Thus, the replication-enhancing effect of the env fragment is not limited to the uAUG mutant, but holds for other replication-impaired viruses as well.

### Enhanced replication is mediated by mutations in different domains of the Env protein.

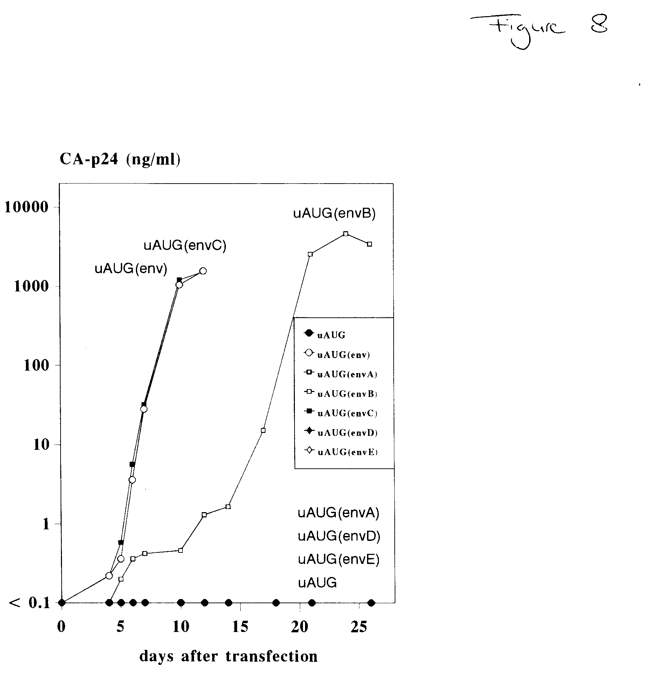

The *env* gene responsible for reversion of the uAUG mutant contains three silent and four non-silent nucleotide changes (Fig. 5). To determine which Env mutation is responsible for the enhanced replication capacity, we cloned five subfragments (Fig. 5, fragments A-E). These fragments were used to replace the corresponding sequences in the original UAUG mutant. Replication of these clones (uAUG_{(envA)}-uAUG_{(envE)}) was assayed by transfection of SupT1 cells (Fig. 8). Two *env* fragments were able to boost replication of the uAUG mutant. Fragment C, which contains a silent and a non-silent (G436R) mutation, dramatically activated the replication potential of the uAUG mutant in SupT1 cells.

The fragment B with the A70T mutation also improved virus replication, but to a lesser extent. The other mutants containing subfragments A,D or E did not replicate, demonstrating that the corresponding mutations are not sufficient to rescue replication of the uAUG mutant. These results show that modification of several Env epitopes increased the HIV-1 replication capacity in SupT1 cells.

### Discussion

To study the translational mechanism of HIV-1, we inserted a translation initiation site in the leader region of the viral RNA to open a short reading frame. This mutation inhibited viral gene expression and a concomitant decrease in virus replication was measured. Upon prolonged culturing of this replication-impaired virus in SupT1 T cells, phenotypic revertants were obtained with greatly increased replication capacity. To our surprise, we found that the additional open reading frame was maintained in the revertant viruses. Further analysis indicated that multiple sequence changes in Env were responsible for the phenotypic reversion. These second-site mutations did not exclusively improve replication of the uAUG mutant, but also of other replication-impaired HIV-1 mutants. Thus, the Env adaptation does not directly overcome the uAUG-specific gene expression defect, but instead leads to a more general optimization of virus replication.

Introduction of the short open reading frame upstream of the HIV-1 cistrons reduced gene expression to 6-18% of the wild-type level. This result is fully consistent with a linear scanning mechanism of translation. The residual production of viral proteins may result either from ribosomes that bypass the upstream start site (leaky scanning) or from ribosomes that resume scanning after translation of the upstream open reading frame (reinitiation). Concluding, ribosomal scanning over the HIV-1 leader RNA is allowed despite the presence of multiple RNA structures. Consistent with this finding, we suggested previously that several hairpin motifs in the HIV-1 RNA leader do not exceed a certain thermodynamic stability (4,8,15). Two additional arguments favour the scanning mechanism. First, a recent study with bicistronic reporter genes indicates that the HIV-1 leader region does not function as an IRES (36).

Second, it is striking that upstream AUG codons are strongly underrepresented in leader regions of natural HIV-SIV isolates. Inspection of the leader RNA of 60 natural isolates revealed only 4 AUGs (38).

One intriguing point that remains unexplained is the persistence of the introduced uAUG mutation in the virus revertants with improved replication potential. We have analyzed many other defective HIV-1 mutants with alterations in leader RNA motifs, but revertants consistently arose by first- or second-site mutations within the mutated motif (9,14,15,29). Here, we have studied three independent reversion events of the uAUG mutant, but the upstream reading frame was maintained in all cases. This is particularly striking because it is relatively simple to inactivate the new AUG codon. It therefore appears that the uAUG mutant is uniquely suited for forced adaptation studies. In prolonged cultures, spontaneously mutated viruses with improved replicative capacity will outcompete the original virus, and the time required for this outgrowth depends on the relative increase in replication rate.

However, the wild-type LAI virus replicates efficiently in many cell types and, although spontaneous mutations may improve its replication capacity, increases in viral fitness will be relatively small and outgrowth of the new variant will be slow. In contrast, such spontaneous sequence changes cause a relatively large increase in fitness of the replication-impaired uAUG mutant, resulting in rapid appearance of adapted HIV-1 variants. Because the uAUG-mutation is stable, yet exhibiting a severe replication defect, this virus is useful for other evolutionary protocols, e.g., to optimize the Env protein for replication on different host cell types.

Adaptation of HIV-1 for replication on T cell lines has been observed previously. For instance, the ELI clone replicates efficiently in PBMC, but poorly in the CEM and H9 T cell lines, and not at all in SupT1 and U937 cells. A revertant virus obtained upon prolonged culturing in H9 cells replicated efficiently in all cell types (40). This expanded host range and increased replicative capacity was conferred by one amino acid change in gpl2O (G427R, comparable position in LAI E434) and two mutations in gp4l (M7V, E49G) (22). Similarly, the macrophagetropic, non-syncytium inducing HIV-1 clone SF162 replicates efficiently in macrophages, but not in T-cell lines. Upon prolonged culturing of this virus in HUT78 T cells, a T-cell line tropic, syncytium-inducing virus was obtained that replicated efficiently in several T-cell lines, but not in macrophages (26). This altered host range was mediated by two amino acid substitutions in the V3 loop of envelope. In our study, two substitutions in the conserved Cl and C4 domains (A70T and G436R) improve HIV-1 replication in SupT1 cells. The evolution of "constant" Env domains during adaptation of HIV-1 LAI to the SupT1 cell line is remarkable if we consider the almost absolute conservation of these two amino acid residues in natural virus' isolates. The A70T mutation in the Cl domain, is not present in 184 natural HIV-1 isolates (38), and only 3 substitutions are observed at this position (181 x A, 1 x D, 1 x E and 1 x P). The G436R mutation in the C4 domain is even more striking because 233 natural isolates reveal an almost absolute conservation of this residue (232 x G, 1 x E).

We are currently testing at what level the modified Env protein does improve replication in the SupT1 cell line. First, it is possible that the Env mutations improve processing of the Env gpl6O precursor protein (30,42).

Second, virus entry may be stimulated by adaptation of the HIV1 Env protein to a SupT1-expressed coreceptor of the chemokine receptor family (1,13,17-19,21). Consistent with this idea, preliminary experiments indicate that the selected Env protein does not support virus replication in other cells (not shown). Obviously, such specialized Env moieties are useful in the design of new retroviral vectors that target specific cell types in gene therapy approaches.

### Legend to the figures.

**Figure 1. Introduction of a translation start site in the leader of the HIV-1 RNA.** A schematic of HIV-1 viral RNA and the introduced sequence changes are shown. An AUG triplet in the context of a consensus translation initiation site was introduced at position +239 in the leader (mutant uAUG). This mutation is positioned upstream of the major splice donor site (SD at position +294), such that all HIV-1 RNAs contain this additional AUG. Nucleotide numbers refer to the position on the genomic RNA transcript; +1 is the capped G residue and the AUG start codon of the *gag* gene is at position +336.

**Figure 2. Western blot analysis of transiently produced viral proteins and viruses.** C33A cells were transfected with wild-type (LAI) and uAUG-mutated (uAUG) proviral constructs. In LAI₍ₑₙᵥ₎ and uAUG₍ₑₙᵥ₎, the *env* coding region was replaced by the revertant *env* fragment of clone uAUGIVb (see Fig. 5). At three days post-transfection, total cellular extracts were prepared and analyzed. Viral proteins were identified with serum of an HIV-1 infected individual. The position of the HIV-1 Gag p55 precursor protein, the MA-CA p4l processing intermediate, and the mature CA p24 protein is indicated on the left. The position of the molecular mass marker proteins (in kilodaltons) is indicated on the right. Lane 1, transfection of the wild-type construct; lanes 2-4, transfection of mutant constructs (indicated on top of the panel); lane 5, mock transfected cells.

**Figure 3. Replication of wild-type and uAUG mutant HIV-1 viruses. Left panel:** SupT1 cells were transfected with wild-type (LAI) and mutant (uAUG) proviral constructs (1 and 5 µg DNA). CA-p24 production was measured in the culture supernatant at several times. Virus stocks of the uAUG mutant and wild-type virus were prepared at 6 and 4 days, respectively, after transfection of 5 µg of DNA.

**Right panel:** The UAUG mutant was cultured for up to 110 days and virus samples were taken at several time points (the day of sampling is indicated). The uAUG mutant and wild-type viruses were derived from the transfection shown in the left panel. The same amounts of virus (normalized by CA-p24 levels) were used to infect fresh SupT1 cells.

**Figure 4. Reversion of the uAUG mutant by second-site mutations. Upper panel:** Schematic of the HIV-1 proviral genome. To identify the mutations that mediate reversion of the uAUG mutant, the proviral DNA present at-day 110 posttransfection (Fig. 3) was PCR-ampliied in five segments (fragments I-V). These PCR fragments were used to replace the corresponding sequences of the original uAUG clone. The restriction sites used and their position on the proviral genome are indicated. The 5' primer used for amplification of fragment I included an *Xba*I site, whereas the 3' primer used for fragment V included an *Aat*II site. **Lower panel:** The replication potential of the uAUG-revertant clones was assayed by transfection of SupT1 cells. For fragment I, IV and V two revertant clones were tested, fragment III was not tested.

**Figure 5. Env protein mutations in revertants of the uAUG virus.** The complete env coding region is shown, with the conserved (C) and variable (V) regions of gpl2O, and the transmembrane domain (TM) of gp4l. The *Sal*I-*Bam*HI fragments of revertant clone uAUG-IVb and two additional revertants (cultures M 15A and M 15B) were sequenced. Silent (open circles) and non-silent (filled circles) mutations are indicated. The mutations were named by appending the amino acid (one-letter code) present in the LAI Env protein, the residue number, and the amino acid present in the revertant at this position. The corresponding nucleotide changes in lVb are: 124T, T-C at nucleotide position 6327 (silent mutation in the vpu gene); A70T, G-A at 6464; Y389Y, C-T at 7423; G436R, GA at 7562; A538A, G-A at 7870; Q557Q, G-A at 7927; A705V, C-T at 8370. In M15A: E32K, G-A at 6350; A55V, C-T at 6420; ViOll, G-A at 6557; P188S, C-T at 6818. In M15B: G385E, G-A at 7410; G436E, G-A at 7563; S470S, C-T at 7666; K507K, G-A at 7777. A preference for G-A and C-T transitions (9 and 5 x out of a total of 15 mutations) in HIV mutation studies was described previously (28). An 104-nt deletion was observed in the U3 region of the LTR of revertant M15A (proviral positions 76-179), which removed part of the *nef* coding region. Deletion of *nef* sequences has been observed previously (7) and does not affect replication of HIV-1 in T-cell lines (16,25,37,43). Because this region was not mutated in the other revertants, it seems unlikely that this U3 deletion mediated the improved replication of the M 15A revertant.

**Figure 6. Env mutations rescue replication of the UAUG virus.** Proviral clones (5 µg) with a wild-type env gene (uAUG and LAI) or the revertant env gene of fragment lVb (uAUG₍ₑₙᵥ₎ and LAI₍ₑₙᵥ₎) were transfected into SupT1 cells. CA-p24 levels were measured in the culture supernatant at several times posttransfection.

**Figure 7. The modified Env protein improves replication of other defective HIV-1 mutants.** The revertant *env* sequence of fragment lVb was introduced into replication-defective HIV-1 clones mutated in either the TAR hairpin (mutant Xho+10; 29) or the polyA hairpin motif (mutants B and B200; 15). These clones are termed: Xho+10₍ₑₙᵥ₎, B₍ₑₙᵥ₎ and B200₍ₑₙᵥ₎, respectively. SupT1 cells were transfected with 0.5 µg of the B, B200 and Xho+10 proviral clones (left panel) and with 5 µg of the Xho+10 clones (right panel). CA-p24 production was measured in the culture supernatant at several time points.

**Figure 8. Different domains of the modified Env protein stimulate virus replication.** Fragment IVb was split into five subfragments (Fig. 5, fragments A-E) that were used to replace the corresponding sequences in the original uAUG mutant. These clones (uAUG_{(envA)}-uAUG_{(envE)}), and the uAUG-mutated proviral clones with a wild-type *env* gene (uAUG) or the complete revertant *env* gene of fragment IVb (uAUG₍ₑₙᵥ₎) were transfected into SupT1 cells (1 µg of DNA). CA-p24 levels were measured in the culture supernatant at several days posttransfection.

### References

1. **Alkhatib,** G., C. **Combadiere,** C. C. **Broder,** Y. Feng, **P.** M. **Murphy,** and E. **A. Berger.** 1996. A RANTES, MIP-la, MIP-lb receptor as a fusion co-factor for macrophage-tropic HIV-1. Science 272:1955-1958.
2. **Auersperg,** N. 1964. Long-term cultivation of hypodiploid human tumor cells. J. Nat. Cancer Inst. 32:135-163.
3. **Belsham,** G. **J. and** N. **Sonenberg.** 1996. RNA-protein interactions in regulation of picornavirus RNA translation. Microbiol. Rev. 60:499-51 1. ,4.
   **Berkhout, B.** 1992. Structural features in TAR RNA of human and simian immunodeficiency viruses: a phylogenetic analysis. Nucleic Acids Res. 20:27-31.
5. **Berkhout, B.** 1996. Structure and function of the Human Immunodeficiency Virus leader RNA. Progr. Nucl. Acid. Res. Mol. Biol. 54:1-34.
6. **Berkhout, B. and B. Klaver.** 1993. In vivo selection of randomly mutated retroviral genomes.Nucleic Acids Res. 21:5020-5024.
7. **Berkhout, B. and B. Klaver.** 1995. Revertants and pseudo-revertants of human immunodeficiency virus type I viruses mutated in the long terminal repeat promoter region. J. Gen. Virol. 76:845-853.
8. **Berkhout, B., B. Klaver, and A. T. Das.** 1995. A conserved hairpin structure predicted for the poly(A) signal of human and simian immunodeficiency viruses. Virol. 207:276-281.
9. **Berkhout, B. and J. L. B. van Wamel.** 1996. Role of the DIS hairpin in replication of human immunodeficiency virus type 1. J. Virol. 70:6723-6732.
10. **Berlioz,** C. **and J.-L. Darlix.** 1995. An internal ribosomal entry mechanism promotes translation of murine leukemia virus gag polyprotein precursors. J. Virol. 69:2214-2222.
11. **Berlioz,** C., C. **Torrent, and J.-L. Darlix.** 1995. An internal ribosomal entry signal in the rat VL30 region of the Harvey murine sarcoma virus leader and its use in dicistronic vectors. J. Virol. 69:6400-6407.
12. **Bieth, E.,** C. **Gabus, and J.-L. Darlix.** 1990. A study of the dimer formation of Rous sarcoma virus RNA and its effect on viral protein synthesis in vitro. Nucleic Acids Res. 18:119-127.
13. **Choe, H., M. Farzan, Y. Sun, N. Sullivan, B. Rollins, P. D. Ponath, L. Wu,** C. **R. Mackay, G. LaRosa, W. Newman, N. Gerard,** C. **Gerard, and J. Sodroski.** 1996. The b-chemokine receptors CCR3 and CCR5 facilitate infection by primary HIV-1 isolates. Cell 85:1135-1148.
14. **Das, A. T., B. Klaver, and B. Berkhout.** 1995. Reduced replication of human immunodeficiency virus type I mutants that use reverse transcription primers other than the natural tRNA(3Lys). J. Virol. 69:3090-3097.
15. **Das, A. T., B. Klaver, B. l. F. Klasens, J. L. B. van Wamel, and B. Berkhout.** 1996. A conserved hairpin motif in the R-U5 region of the human immunodeficiency virus type-I RNA genome is essential for replication. J. Virol. 71, 2346-2356
16. **de Ronde, A., B. Klaver, W. Keuien, L. Smit, and J. Goudsmft.** 1992. Natural HIV-1 NEF accelerates virus replication in primary human lymphocytes. Virol. 188:391-395.
17 **Deng, H., R. Liu, W. Ellmeier,** S. **Choe, D. Unutmaz, M. Burkhart, P. Di Marzio,** S. **Marmon, R.** E. **Sutton, C.** M. **Hill, C. B. Davis, S. Peiper, T. J. Schall, D. R. Liffman, and N. R. Landau.** 1996. Identification of a major co-receptor for primary isolates of HIV-1. Nature 381:667-673.
18. **Doranz, B. J., J. Rucker, Y. Yi, R. J. Smyth, M. Samson, S. Peiper, M. Parmentier, R.** G. **Coliman, and R. W. Doms.** 1996. A dual-tropic primary HIV-1 isolate that uses fusin and the beta-chemokinereceptors CKR-5, CKR-3, and CKR-2beta as fusion cofactors. Cell **85:1149-1158.**
19. **Dragic,** T., **V. Litwin, G. P. Allaway, S. R. Martin, Y. Huang, K. A. Nagashima,** C. **Cayanan, P. J. Maddon, R. A. Koup, J. P. Moore, and W. A. Paxton.** 1996. HIV-1 entry into CD4+ cells is mediated by the chemokine receptor CC-CKR5. Nature 381:667-673.
20. **Edery, l. R., R. Petryshyn, and N. Sonenberg.** 1989. Activation of double-stranded RNA dependent kinase (dsl) by the TAR region of HIV-1 MRNA: a novel translational control mechanism. Cell **566303-312..**
21. **Feng,** Y., C. C. **Broder,, P. E. Kennedy, and E. A. Berger.** 1996. HIV- I entry cofactor: functional CDNA cloning of a seven-transmembrane, G protein couplked receptor. Science 272:872-877.
22. **Fujita, K., J. Silver, and K. Peden.** 1992. Changes in both gpl2O and gp4l can account for increased growth potential and expanded host range of human immunodeficiency virus type 1. J. Virol. 66:4445-4451.
23. **Futterer, J., Z. Kiss-Laszio, and T. Hohn.** 1993. Nonlinear ribosome migration on cauliflower mosaic virus 355 RNA. Cell 73:789-802. 24. **Geballe, A. P. and M. K. Gray.** 1992. Variable inhibition of cell-free translation by HIV-1 transcript leader sequences. Nucleic Acids Res. 20:4291-4297. 25. **Gibbs, J.** S., **D. A. Regier, and R.** C. **Desrosiers.** 1994. Construction and in vitro properties of HIV-1 mutants with deletions in "nonessential" genes. AIDS Res. Hum. Retroviruses 10:343-350.
26. **Harrowe, G. and C. Cheng-Mayer.** 1995. Amino acid substitutions in the V3 loop are responsible for adaptation to growth in transformed T-cell lines of a primary human immunodeficiency virus type 1. Virol. 210:490-494.
27. **Honda,** M., **E. A. Brown, and** S. M. **Lemon.** 1996. Stability of a stem-loop involving the initiator AUG controls the efficiency of internal initiation of translation on hepatitis C virus RNA. RNA 2:955-968.
28. **Keuien, W.,** C. **Boucher, and B. Berkhout.** 1996. Nucleotide substitution patterns can predict the requirements for drug-resistance of HIV-1 proteins. Antiviral Res. 31:45-57.
29. **Klaver, B. and B. Berkhout.** 1994. Evolution of a disrupted TAR RNA hairpin structure in the HIV-1 virus. EMBO J. 13:2650-2659.
30. **koken,** S. E. C. **and B. Berkhout.** 1994. Intracellular processing of the Env protein of the human immunodeficiency virus type I is regulated in a cell-type dependent manner. In: Transcriptional regulation ofthe human immunodeficiency virus, S. E. C. Koken, P. 25-43. PhD Thesis, University of Amsterdam, Amsterdam.
31. Kozak, M. 1986. Point mutations define a sequence flanking the AUG initiator codon that modulates translation by eukaryotic ribosomes. Cell 44:283-292.
32. **Kozak,** M. 1986. Influence of MRNA secondary structure on initiation by eukaryotic ribosomes. Proc. Natl. Acad. Sci. USA 83:2850-2854.
33. **Kozak,** M. 1989. Circumstances and mechanisms of inhibition of translation by secondary structure in eucaryotic mRNAs. Mol. Cell Biol. 9:5134-5142.
34. **Kozak,** M. 1989. The scanning model for translation: an update. J. Cell Biol. 108:229-241.
35. **Kunkel, T. A., J. D. Roberts, and R. A. Zakour.** 1987. Rapid and efficient site-specific mutagenesis without phenotypic selection, p. 367-382. In R. Wu and L. Grossman (eds.), Methods in Enzymology. Academic Press, San Diego.
36. Miele, G., **A. Mouland, G. P. Harrison,** E. **Cohen, and A. M. Lever.** 1996. The human immunodeficiency virus type 1 5' packaging signal structure affects translation but does not function as an internal ribosome entry site structure. J. Virol. 70:944-951.
37. **Miller, M. D., M. T. Warmerdam, l. Gaston, W.** C. **Greene, and M. B. Feinberg.** 1994. The human immunodeficiency virus-I nef gene product: a positive factor for viral infection and replication in primary lymphocytes and macrophages. J. Exp. Med. **179:101-113.**
38. **Myers, G., B. Korber, B. H. Hahn, K.-T. Jeang, J. H. Mellors, F. E. McCutchan, L. E. Henderson, and G. N. Paviakis.** 1995. Human retroviruses and AIDS. A compilation and analysis of nucleic acid and amino acid sequences. Theoretical Biology and Biophysics Group, Los Alamos National Laboratory, Los Alamos, New Mexico.
39. **Parkin,** N. **T.,** E. **A. Cohen, A. Darveau,** C. **Rosen, W. Haseftine, and N. Sonenberg.** 1988. Mutational analysis of the 5' noncoding region of human immunodeficiency virus type 1: effects of secondary structure. EMBO J. 7:2831-2837.
40. **Peden, K.,** M. **Emerman, and** L. **Montagnier.** 1991. Changes in growth properties on passage in tissue 'culture bf viruses derived from infectious molecular' clones of HIV-ILI, HIV-1., and HIV-1 **ELI** - Virol. 185:661-672.
41. **SenGupta, D**. N., **B. Berkhout, A. Gatignol, A. M. Zhou, and R.** H. **Silverman.** 1990. 'Direct evidence for translational regulation by leader RNA and Tat protein of human immunodeficiency virus type 1. Proc. Natl. Acad. Sci. USA 87:7492-7496.
42. **Shahabuddin,** M., G. **Bentsman, B. Voisky, l. Rodriguez, and D. Volsky,J.** 1996. A mechanism of restricted human immunodeficiency virus type I expression in human glial cells. J. Virol. 70:7992-8002.
43. **Spina,** C. **A., T. J. Kwoh,** M. Y. **Chowers, J. C. Guatelli, and D. D. Richman.** 1994. The importance of nef in the induction of human immunodeficiency virus type I replication from primary quiescent CD4 lymphocytes. J. Exp. Med. 179:115-123.
44. **Vagner,** S., **A. Waysbort,** M. **Marenda,** M.-C. **Gensac,** F. **Amalric,** and A.-C. **Prats.** 1995. Alternative translation initiation of the Moloney murine leukemia virus MRNA controlled by internal ribosome entry involving the p57/PTB splicing factor. J. Biol. Chem. 270:20376-20383.
45. **Willey, R.** L., **D. H. Smith,** L. **A. Lasky, T. S. Theodore, P.** L. **Earl, B. Moss, D. J. Capon, and** M. **A. Martin.** 1988. In vitro mutagenesis identifies a region within the envelope gene of the human immuno deficiency virus that is critical for infectivity. J. Virol. 62:139-147.

**Table 1**

| **Virus production in C33A** cells. | | |
|---|---|---|
| | CA-p24' ng/mi | RTa counts x 10' |
| LAI | 1674(100) | 34622 (100) |
| UAUG | 176(11) | 6228 (18) |
| LAI(env)' | 1559(93) | 29981 (87) |
| uAUG(env)' | 96 (6) | 3002 (9) |

_ The CA-p24 concentration and RT activity were determined in the culture supernatant at 3 days posttransfection.
_ In uAUG(env) and LAI(env), the env coding region was replaced by the revertant env fragment of clone uAUG-lVb (see Fig. 5).

## Claims

1. A method for producing a retrovirus or a recombinant virus derived therefrom having an altered tropism, whereby the genome of the virus is modified so that at least one of its translation initiation sites is at least partially functionally inhibited.

2. A method according to claim 1 wherein the retrovirus is an immunodeficiency virus.

3. A method according to claim 2 wherein the immunodeficiency virus is a human or simian immunodeficiency virus.

4. A method according to anyone of claims 1-3 whereby the functional inhibition is accomplished by introduction of a translation initiation site upstream of the translation initiation site of the wild-type virus.

5. A method according to claim 4 whereby said introduced translation initiation site is followed by a short open reading frame.

6. A method according to anyone of the aforegoing claims which comprises infecting host cells with the modified virus and culturing said infected cells for a sufficient time for the modified virus to circumvent the inhibition introduced by the modification and thereby alter its tropism.

7. A method according to claim 6 whereby the alteration of tropism is a narrowing of the host cell range.

8. A method according to claim 6 or 7 whereby the alteration of tropism includes an enhancement of the infectiousness of the modified virus for a certain subset of host cells.

9. A modified retrovirus or a recombinant viral vector derived therefrom obtainable by a method according to anyone of the aforegoing claims.

10. A modified retrovirus according to claim 9 which comprises in its genome a heterologous nucleic acid sequence.

11. A modified retrovirus according to claim 10, whereby the heterologous nucleic acid sequence is a nucleic acid sequence of therapeutic interest.

12. A modified retrovirus according to claim 10 or 11 whereby the heterologous nucleic acid sequence encodes a protein or an antisense nucleic acid molecule.

13. A recombinant vector for preparing a modified virus according to claims 10-12, comprising a provirus DNA sequence having a modification resulting in a functional at least partial inhibition of at least one translation initiation site of the original retrovirus genome.

14. A recombinant vector acccording to claim 13 whereby the modification comprises an additional translation initiation site upstream of the original translation initiation site.

15. A recombinant vector according to claim 14 whereby the additional translation initiation site is followed by a short open reading frame.

16. A recombinant vector according to anyone of claims 13-15 whereby a heterologous DNA sequence is present within the provirus sequence.

17. A recombinant vector according to claim 16 whereby said heterologous sequence encodes a protein or an antisense molecule.

18. A modified retrovirus according to anyone of claims 9-12 or a recombinant vector according to any one of claims 13-17 for use as a pharmaceutical.

19. A modified retrovirus according to anyone of claims 9-12 or a recombinant vector according to any one of claims 13-17 for use in the preparation of a pharmaceutical for gene therapy.

20. A method for producing a recombinant vector encoding a recombinant retrovirus having modified tropism, comprising subjecting a retrovirus to a method according to anyone of claims 1-8, culturing said retrovirus in a host cell to allow for the virus to circumvent the introduced inhibition, identifying the sequence which was mutated to circumvent said inhibition and introducing said mutation in another vector based on a retrovirus or provirus of a retrovirus.

21. A method according to claim 20 whereby the other retrovirus was not subjected to a method according to claims 1-8.

22. A method according to claim 20 or 21 whereby the other virus is a recombinant virus comprising a heterologous nucleic acid sequence.

23. A retrovirus or provirus obtainable by a method according to claim 20-22.

24. A retrovirus or provirus according to claim 23 for use as a pharmaceutical.

25. A retrovirus or provirus according to claim 23 for use in the preparation of a pharmaceutical for gene therapy.
